# EUROPEAN PATENT APPLICATION

(11) **EP 3 398 512 A1**
(43) Date of publication of application: **07.11.2018**
(21) Application number: 16881450.7
(22) Date of filing: 09.03.2016
(51) Int. Cl.: A61B 5/07

(54) **DETECTION SYSTEM, RECEIVER, AND DETECTION METHOD**

(30) Priority: 28.12.2015 JP 2015255666
(71) Applicant: Tohoku University, Sendai-shi, Miyagi 980-8577 (JP)
(72) Inventor: NAKAMURA, Tsutomu, Sendai-shi Miyagi 980-8577 (JP); YOSHIDA, Shinya, Sendai-shi Miyagi 980-8577 (JP); MIYAGUCHI, Hiroshi, Sendai-shi Miyagi 980-8577 (JP)
(74) Representative: Hoffmann Eitle
(86) International application number: PCT/JP2016/057272
(87) International publication number: WO 2017/115470

(57) **Abstract**

A detecting system (1) includes a sensor (10) and a receiving device (20). The sensor (10) is introduced into a living body through a mouth of the living body, transmits time-point related information associated with a plurality of time points different from one another, detects a physical amount in the living body, and transmits detection information representing the detected physical amount at each of the plurality of time points. The receiving device (20) receives the time-point related information transmitted by the sensor (10) and is on standby for receiving the detection information transmitted by the sensor (10) on the basis of the plurality of time points associated with the received time-point related information.

## Description

### TECHNICAL FIELD

The present invention relates to a detecting system, a receiving device, a method for detecting.

### BACKGROUND ART

A known detecting system includes a sensor that is introduced into a living body through the mouse, that detects a physical amount in the living body, and that transmits detection information representing the detected physical amount, and a receiving device that receives the detection information transmitted by the sensor (see, for example, Patent Literature 1). The sensor receives control information representing a periodic cycle from the receiving device and transmits the detection information on the basis of the periodic cycle represented by the received control information.

### PRIOR ART DOCUMENTS

### Patent Documents

[Patent Document 1] International Publication Pamphlet No. WO 2010/147175

### SUMMARY OF THE INVENTION

### PROBLEMS TO BE SOLVED BY THE INVENTION

In the above detecting system, the sensor consumes electric power while being on standby for receiving the control information transmitted by the receiving device. Consequently, the detecting system tends to consume a large amount of electric power at the sensor (namely, tends to have a large amount of consumption electric power).

Hence, it is thought that the detecting system is configured not to transmit the control information from the receiving device to the sensor. However, providing that the periodic cycle that is used by the sensor to transmit the detection information is different with each sensor, the receiving device may be incapable of specifying the periodic cycle of the sensor introduced in the living body. This tends to unnecessarily prolong the time for which the receiving device is on standby for receiving the detection information transmitted by the sensor, leading to increasing the consumption electric power of the receiving device.

One of the objects of the present invention is to reduce the consumed electric power.

### MEANS TO SOLVE THE PROBLEM

According to an aspect, the detecting system includes: a sensor that is introduced into a living body through a mouth of the living body, that transmits time-point related information associated with a plurality of time points different from one another, that detects a physical amount in the living body, and that transmits detection information representing the detected physical amount at each of the plurality of time points; and a receiving device that receives the time-point related information transmitted by the sensor and that is on standby for receiving the detection information transmitted by the sensor on the basis of the plurality of time points associated with the received time-point related information.

As another aspect, a receiving device includes: a receiver that receives time-point related information and detection information that are transmitted by a sensor, the sensor being introduced into a living body through a mouth of the living body, detecting a physical amount in the living body, and transmitting the detection information representing the detected physical amount at each of a plurality of time points; and a controller that controls the receiver to be on standby for receiving the detection information transmitted by the sensor on the basis of the plurality of time points associated with the received time-point related information.

As an additional aspect, a method for detecting includes: introducing a sensor into a living body through a mouth of the living body; at the sensor, transmitting time-point related information associated with a plurality of time points different from one another; at a receiving device, receiving the time-point related information transmitted by the sensor; at the sensor, detecting a physical amount in the living body; at the sensor, transmitting detection information representing the detected physical amount at each of the plurality of time points; and at the receiving device, being on standby for receiving the detection information transmitted by the sensor on the basis of the plurality of time points associated with the received time-point related information.

### Effect of Invention

The consumption electric power can be reduced.

### BRIEF DESCRIPTION OF DRAWINGS

FIG. 1 is a block diagram schematically illustrating the configuration of a detecting system according to a first embodiment;
FIG. 2 is a block diagram schematically illustrating the configuration of a sensor of FIG. 1;
FIG. 3 is a block diagram schematically illustrating the configuration of a receiving device of FIG. 1;
FIG. 4 is a block diagram schematically illustrating the configuration of a server of FIG. 1;
FIG. 5 is a block diagram schematically illustrating the function of a sensor of FIG. 1;
FIG. 6 is a block diagram schematically illustrating the function of a receiving device of FIG. 1;
FIG. 7 is a block diagram schematically illustrating the function of a server of FIG. 1;
FIG. 8 is a sequence diagram illustrating an example of operation in a detecting system of FIG. 1;
FIG. 9 is a flow chart denoting a process performed by a sensor of FIG. 1;
FIG. 10 is a flow chart denoting a process performed by a receiving device of FIG. 1;
FIG. 11 is a flow chart denoting a process performed by a server of FIG. 1;
FIG. 12 is a flow chart denoting a process performed by a server of FIG. 1;
FIG. 13 is a flow chart denoting a process performed by a receiving device of FIG. 1;
FIG. 14 is a flow chart denoting a process performed by a server of FIG. 1;
FIG. 15 is a block diagram schematically illustrating the function of a receiving device according to a second embodiment;
FIG. 16 is a block diagram schematically illustrating the function of a server of the second embodiment;
FIG. 17 is a sequence diagram illustrating an example of operation in a detecting system of the second embodiment;
FIG. 18 is a flow chart denoting a process performed by a receiving device of the second embodiment;
FIG. 19 is a flow chart denoting a process performed by a receiving device of the second embodiment; and
FIG. 20 is a flow chart denoting a process performed by a server of the second embodiment.

### EMBODIMENTS TO CARRY OUT THE INVENTION

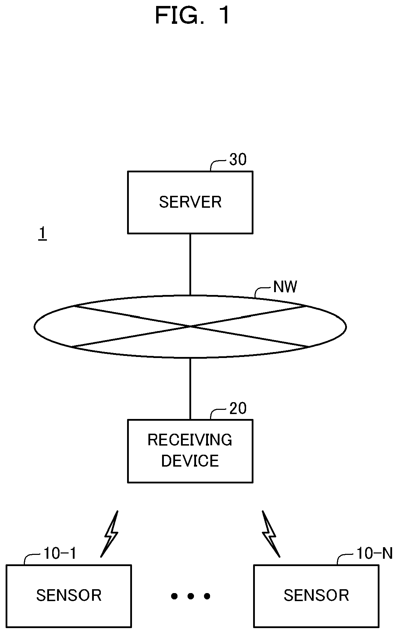

Hereinafter, description will now be made in relation to embodiments of a detecting system, a receiving device, and a method for detecting of the present invention by referring to FIGs. 1-20.

### <first embodiment>

### (configuration)

As illustrated in FIG. 1, a detecting system 1 of the first embodiment includes N sensors 10-1,..., 10-N, a receiving device 20, and a server 30. In this example, N represents an integer of one or more. Hereinafter, when a sensor 10-n is not discriminated from the remaining sensors, a sensor 10-n may be represented by the sensor 10. The symbol n represents an integer of from one to N.

The receiving device 20 is communicably connected to the server 30 via a communication network NW. In this example, the communication path between the receiving device 20 and the server 30 is at least one of a wireless communication path for wireless communication and a wired communication path for wired communication. For example, the receiving device 20 and the server 30 communicate with each other in conformity with a predetermined communication scheme.

The communication scheme used in this example is Long Term Evolution (LTE). Alternatively, the communication scheme may be LTE-Advanced, Worldwide Interoperability for Microwave Access (WiMAX), 3rd Generation (3G), 2nd Generation (2G), or the like.

### (configuration: sensor)

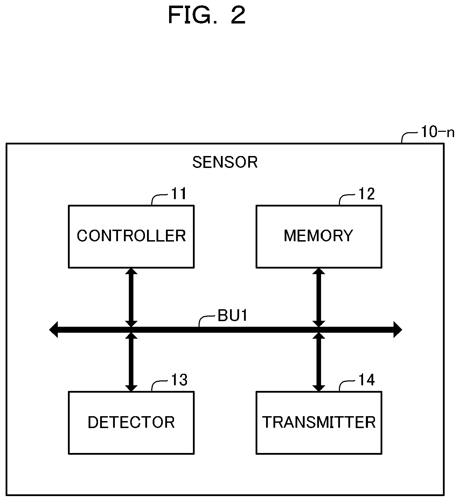

As illustrated in FIG. 2, a sensor 10-n includes a controller 11, a memory 12, a detector 13, and a transmitter 14 that are connected to one another via a bus BU1. In this example, at least part of the sensor 10-n is a Large Scale Integration circuit (LSI). Alternatively, at least part of the sensor 10-n may be a programmable logic circuit such as a Programmable Logic Device (PLD) or a Field-Programmable Gate Array (FPGA).

The controller 11 controls the memory 12, the detector 13, and the transmitter 14 by executing a program stored in the memory 12. Thereby, the controller 11 achieves the following functions.

The memory 12 readably and writably stores therein information.

The controller 11 may include a Central Processing Unit (CPU), a Micro Processing Unit (MPU), or a Digital Signal Processor (DSP). The memory 12 may include a Random Access Memory (RAM), a semiconductor memory, or an organic memory.

The detector 13 detects a physical amount. Specifically, the detector 13 of this example detects the physical amount by converting the physical amount into a signal value (e.g., the value of an electric signal). Examples of a physical amount is temperature, pH, acceleration, pressure, and level of an object. Example of the object is a digestive fluid (e.g., gastric fluid, intestinal fluid, or pancreatic fluid), blood, flora, or infectious material (e.g., bacteria, or virus). A level of the object of a predetermined threshold or more can be understood that the object is present. For example, acceleration or pressure may be used for detecting peristalsis of the alimentary canal.

The detector 13 further detects the start of using the sensor 10-n.

In this example, the detector 13 detects the start of using the sensor 10-n when the sensor 10-n is introduced into a living body (i.e., the alimentary canal of the human) through the mouth. The detector 13 may detect introduction into a living body through the mouth by detecting liquid, or may detect ambient temperature and detect, when the detected temperature is a threshold or higher, introduction into a living body through the mouth.

If the sensor 10-n is stored in a container, the detector 13 may detect the start of using the sensor 10-n when the sensor 10-n is ejected from the container. In this case, the detector 13 may detect light intensity and detect, when the detected light intensity is a threshold or higher, the ejection of the sensor 10-n from the container. Alternatively, if the detector 13 is stored in the container in contact with part of the container, the detector 13 may detect, when detecting departing of the part of the container from the sensor 10-n, the ejection of the sensor 10-n from the container.

The transmitter 14 includes an antenna, and wirelessly transmits a signal via the antenna through a predetermined transmission process. In this example, the transmission process includes a modulation conforming to a predetermined modulation scheme which is exemplified by an analog modulation or a digital modulation.

If an analog modulation is adopted, examples of the modulation scheme of the analog modulation may be Amplitude Modualtion (AM), Frequency Modulation (FM), and Phase Modulation (PM).

If a digital modulation is adopted, the transmission process may include conversion from an analog signal to a digital signal (i.e., Analog-to-Digital (AD) conversion). If a digital modulation is adopted, examples of the modulation scheme of the digital modulation may be Amplitude Shift Keying (ASK), Phase Shift Keying (PSK), and Frequency Shift Keying (FSK).

The transmission process may include encoding using an error correcting code. Examples of the error correcting code may be a convolutional code, a turbo code, and a Low-Density Parity-Check Code (LDPC).

### (configuration: receiving device)

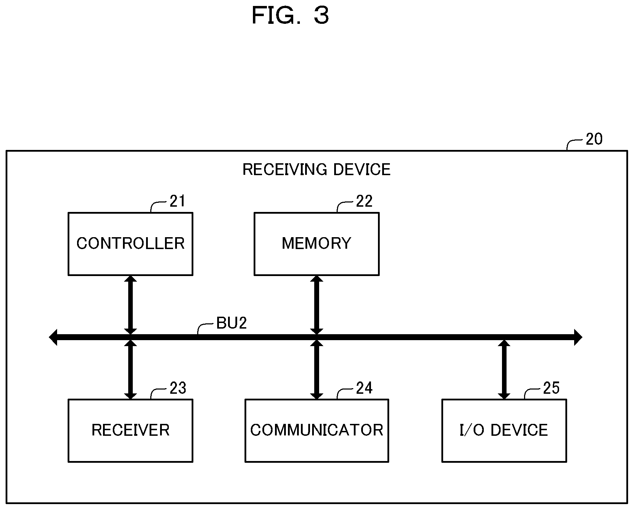

As illustrated in FIG. 3, the receiving device 20 includes a controller 21, a memory 22, a receiver 23, a communicator 24, and an I/O device 25 that are connected to one another via a bus BU2. In this example, at least part of the receiving device 20 is an LSI circuit. Alternatively, at least part of the receiving device 20 may be a programmable logic circuit.

The controller 21 controls the memory 22, the receiver 23, the communicator 24, and the I/O device 25 by executing a program stored in the memory 22. Thereby, the controller 21 achieves the following functions.

The memory 22 readably and writably stores therein information.

The controller 21 may include a CPU, an MPU, or a DSP. The memory 22 may include a RAM, a semiconductor memory, an organic memory, a Hard Disk Drive (HDD), or a Solid State Drive (SSD). The memory 22 may include a recording medium and a reader capable of reading information from the recording medium. Examples of the recoding medium are a flexible disk, an optical disk, a magneto-optical disk, and a semiconductor memory.

The receiver 23 includes an antenna, and wirelessly receives a signal via the antenna through a predetermined reception process. In this example, the reception process includes a demodulation conforming to a demodulation scheme corresponding to the above modulation scheme. Furthermore, the reception process may include an error correcting process using an error correcting code.

The receiver 23 switches the state thereof between a standby state and a non-standby state. In the standby sate, the receiver 23 carries out the reception process. In other words, the receiver 23 is standby for the reception in the standby state.

In contrast, in the non-standby state, the receiver 23 does not carry out the reception process. In other words, the receiver 23 is not on standby for the reception in the non-standby state.

In this example, when the receiver 23 is in the non-standby state, the receiving device 20 stops supplying the receiver 23 with electric power. Alternatively, when the receiver 23 is in the non-standby state, the receiving device 20 may reduce the electric power supplied to the receiver 23 as compared with the electric power supplied to the receiver 23 when the receiver 23 is in the standby state.

The communicator 24 includes an antenna. The communicator 24 wirelessly transmits a signal via the antenna through a transmission process conforming to the above communication scheme. Furthermore, the communicator 24 wirelessly receives a signal via the antenna through a reception process conforming to the above communication scheme.

The I/O device 25 inputs information from the exterior of the receiver 20 into the receiving device 20.
In this example, the I/O device 25 include a key-type button and may further include a microphone. Furthermore, the I/O device 25 outputs information to the outside of the receiving device 20. In this example, the I/O device 25 includes a display, and may further include a speaker. Alternatively, the I/O device 25 may include a touch-panel display.

### (configuration: server)

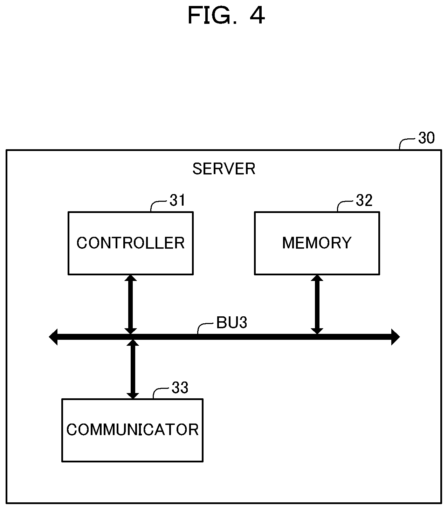

As illustrated in FIG. 4, the server 30 includes a controller 31, a memory 32, and a communicator 33 that are connected to one another via a bus BU3. In this example, at least part of the server 30 is an LSI circuit.
Alternatively, at least part of the server 30 may be a programmable logic circuit.

The controller 31 controls the memory 32 and the communicator 33 by executing a program stored in the memory 32. Thereby the controller 31 achieves the following functions.

The memory 32 readably and writably stores therein information.

The controller 31 may include a CPU, an MPU, or a DSP. The memory 32 may include a RAM, a semiconductor memory, an organic memory, a HDD, or an SSD. The memory 22 may include a recording medium and a reader capable of reading information from the recording medium. Examples of the recoding medium are a flexible disk, an optical disk, a magneto-optical disk, and a semiconductor memory.

The communicator 33 includes an antenna. The communicator 33 wirelessly transmits a signal via the antenna through a transmission process conforming to the above communication scheme. Further, the communicator 33 wirelessly receives a signal via the antenna through a reception process conforming to the above communication scheme.

### (function: sensor)

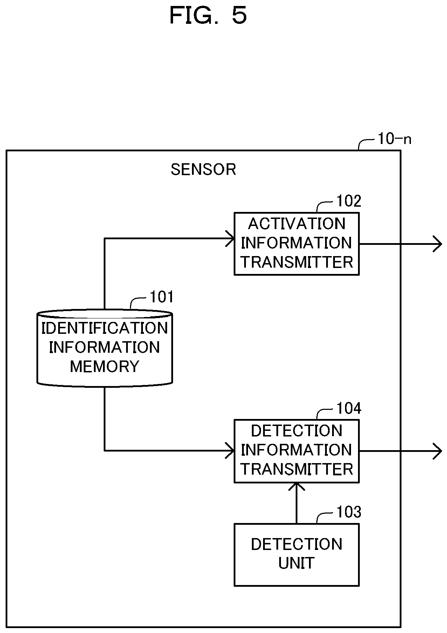

As illustrated in FIG. 5, the sensor 10-n has the functions of an identification information memory 101, and activation information transmitter 102, a detection unit 103, and a detection information transmitter 104.

The identification information memory 101 stores identification information and transmitting time-point information beforehand using the memory 12.

The identification information identifies the sensor 10-n among the N sensors 10-1,..., 10-N. In this example, the identification information for the sensor 10-n is peculiar to the sensor 10-n.

In this example, the identification information includes type identification information that identifies the type of sensor 10-n and type-basis sensor identification information that identifies the sensor 10-n from the sensors 10 of the same type among the N sensors 10-1,..., 10-N. For example, the type of sensor 10 may correspond to at least one of the type of physical amount to be detected by the sensor 10 and the manufacturer of the sensor 10.

The transmitting time-point information represents multiple transmitting time points different from one another. A transmitting time point is a time point at which detection information that is to be described below is transmitted. In this example, the transmitting time point is set for each type of sensor 10. In this example, the transmitting time-point information includes information representing multiple transmitting standby times different from one another. A transmitting time point of this example corresponds to a time point at which a transmitting standby time has passed since a predetermined reference time point. The reference time point is a time point at which activation information is transmitted. Alternatively, the reference time point may be a time point at which the start of using the sensor 10-n is detected. For example, multiple transmitting time points represented by the transmitting time-point information are set to have regular or irregular intervals.

In cases where the detection unit 103 detects the start of using the sensor 10-n, the activation information transmitter 102 transmits activation information to the receiving device 20 using the transmitter 14. The activation information includes the identification information stored in the identification information memory 101. The activation information represents the start of using the sensor 10-n. The activation information may be referred to as using started information. The activation information of this example corresponds to time-point related information.

The detection unit 103 detects the start of using the sensor 10-n using the detector 13. In addition, the detection unit 103 detects a physical amount by converting the physical amount into a signal value using the detector 13 at each of the multiple transmitting time points represented by the transmitting time-point information stored in the identification information memory 101. The detection unit 103 may detect the physical amount each time a predetermined detecting period passes.

The detection information transmitter 104 transmits detection information representing the signal value obtained by the conversion by the detection unit 103 to the receiving device 20 using the transmitter 14 at each of the multiple transmitting time points represented by the transmitting time-point information stored in the identification information memory 101. The detection information of this example includes identification information stored in the identification information memory 101. Alternatively, the detection information does not have to include identification information.

### (function: receiving device)

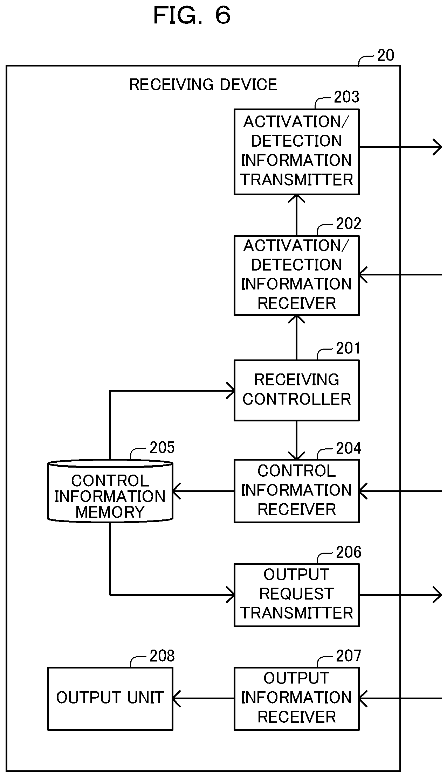

As illustrated in FIG. 6, the receiving device 20 has the functions of a receiving controller 201, an activation/detection information receiver 202, an activation/detection information transmitter 203, a control information receiver 204, a control information memory 205, an output request transmitter 206, an output information receiver 207, and an output unit 208. In this example, the activation/detection information receiver 202 corresponds to the receiver and the receiving controller 201 corresponds to the controller.

The receiving controller 201 controls the state of the receiver 23 to be the standby state or the non-standby state.

The receiving controller 201 controls, when the receiving device 20 is activated, the state of the receiver 23 to be the standby state.

When the receiver 23 is in the standby state, the activation/detection information receiver 202 receives the activation information transmitted by the sensor 10-n using the receiver 23.

When the activation/detection information receiver 202 receives the activation information, the activation/detection information transmitter 203 transmits the received activation information to the server 30 using the communicator 24.

The control information receiver 204 receives control information transmitted by the server 30 using the communicator 24. The control information of this example contains the identification information and the transmitting time-point information.

When the control information receiver 204 receives the control information, the control information memory 205 stores the received control information using the memory 22.

The receiving controller 201 determines multiple receiving standby time periods on the basis of the control information stored in the control information memory 205 and the time point at which the activation/detection information receiver 202 receives the activation information (in other words, the time point of receiving the activation information).

The receiving controller 201 may uses a time point at which the activation information is transmitted as the time point at which the activation information is received. In this case, the activation information may include information representing the time point at which the activation information is transmitted.

In this example, the multiple receiving standby time periods include time points (i.e., estimated reception time points) at which multiple transmitting standby times represented by the transmitting time-point information included in the control information have passed since the time point of receiving the activation information. In this example, the lengths of the receiving standby time periods are determined in advance.

The time point at which each receiving standby time period starts may be referred to as a standby start time point while the time point at which each receiving standby time period finishes may be referred to as a standby finish time point. In this example, determining the receiving standby time periods includes determining the respective standby start time points and determining the respective standby finish time points.

For example, in the receiving standby time period, the length of from the standby start time point to the estimated reception time point may be the same as the length of from the estimated reception time point to the standby finish time point. Alternatively, in the receiving standby time period, the length of from the standby start time point to the estimated reception time point may be different from the length of from the estimated reception time point to the standby finish time point.

The receiving controller 201 controls the state of the receiver 23 to be the standby state each time the standby start time point comes.

When the receiver 23 is in the standby state, the activation/detection information receiver 202 receives detection information transmitted by the sensor 10-n using the receiver 23.

Each time the activation/detection information receiver 202 receives the detection information, the activation/detection information transmitter 203 transmits the received detection information to the server 30 using the communicator 24.

Each time the activation/detection information receiver 202 receives the detection information, the receiving controller 201 controls the state of the receiver 23 to be the non-standby state. Furthermore, each time the determined standby finish time point comes, the receiving controller 201 controls the state of the receiver 23 to be the non-standby state.

The output request transmitter 206 transmits an output request to the server 30 using the communicator 24 in response to information input by the user of the receiving device 20 via the I/O device 25. In this example, the output request is a request for output information that is to be described below, and includes the identification information stored in the control information memory 205. The output request transmitter 206 may transmit the output request to the server 30 each time a predetermined output period passes.

The output information receiver 207 receives output information transmitted by the server 30 using the communicator 24.

When the output information is received by the output information receiver 207, the output unit 208 outputs the received output information using the I/O device 25 (e.g., presents the received output information on a display) .

In this example, as described above, each time the receiving device 20 receives the detection information from the sensor 10-n, the receiving device 20 transmits the received detection information to the server 30. Each time the receiving device 20 repeats the reception of the detection information from the sensor 10-n for a predetermined repeat number of two or more, the receiving device 20 may transmit the repeated number of pieces of the detection information received therein to the server 30 in a lump.

### (function: server)

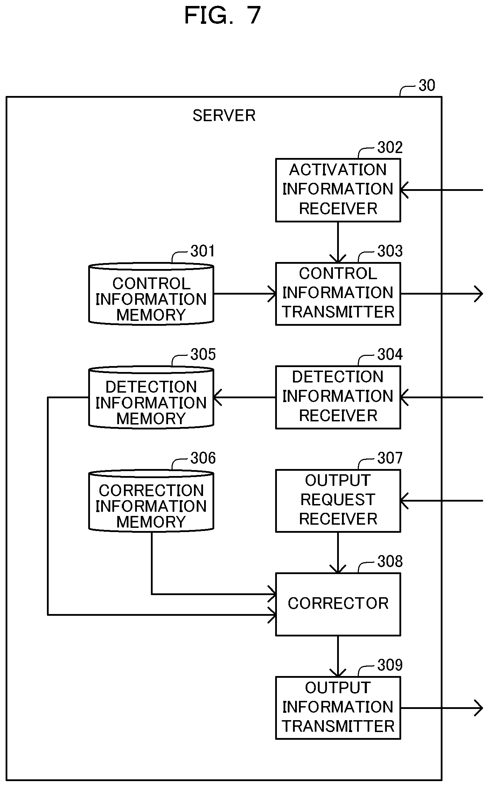

As illustrated in FIG. 7, the server 30 has the functions of a control information memory 301, an activation information receiver 302, a control information transmitter 303, a detection information receiver 304, a detection information memory 305, a correction information memory 306, an output request receiver 307, a corrector 308, and an output information transmitter 309.

The control information memory 301 stores, for each type of sensor 10, type identification information that identifies the type and the transmitting time-point information representing multiple transmitting time points set for the type of sensor 10 in association with each other using the memory 32.

The activation information receiver 302 receives the activation information transmitted by the receiving device 20 using the communicator 33.

When the activation information receiver 302 receives the activation information, the control information transmitter 303 obtains the transmitting time-point information that is stored in the control information memory 301 in association with the type identification information included in the identification information included in the received activation information. Furthermore, the control information transmitter 303 transmits the control information including the obtained transmitting time-point information and the identification information included in the received activation information to the receiving device 20 using the communicator 33.

The detection information receiver 304 receives the detection information transmitted by the receiving device 20 using the communicator 33.

Each time the detection information receiver 304 receives the detection information, the detection information memory 305 stores the received detection information using the memory 32.

The correction information memory 306 stores, for each sensor 10, identification information that identifies the sensor 10 and correction information that is used for correcting the relationship between a signal value and a physical amount of the sensor 10 in association with each other using the memory 32. The correction here may be referred to as calibration. For example, the correction information is set on the basis of the results of test, inspection, or evaluation conducted when the sensor 10 is manufactured.

The output request receiver 307 receives the output request transmitted by the receiving device 20 using the communicator 33.

When the output request receiver 307 receives the output request, the corrector 308 obtains the detection information including the identification information included in the received output information among the detection information stored in the detection information memory 305.

Furthermore, the corrector 308 obtains the correction information stored in the correction information memory 306 in association with the type identification information included in the identification information contained in the received output request. The corrector 308 corrects the relationship between the signal value represented by the obtained detection information and the physical amount on the basis of the obtained correction information. The corrector 308 converts a signal value represented by the obtained detection information into a physical amount in conformity with the corrected relationship.

For example, when the correction information represents a correction amount to a physical amount, the corrector 308 may convert the signal value represented by the detection information into a physical amount in accordance with the relationship predetermined for the type of sensor 10 and may add the correction amount to the converted physical amount.

Alternatively, the receiving device 20 may perform the correction on the basis of the correction information in place of the server 30 in the detecting system 1. In this case, the server 30 may transmit the correction information stored in the correction information memory 306 and the detection information stored in the detection information memory 305 to the receiving device 20.

The output information transmitter 309 generates output information based on the physical amount converted by the corrector 308. For example, the output information includes information representing multiple time points and physical amounts detected at the respective time points.
The output information also includes information representing, for example, the statistics (e.g., average or variance) of the multiple physical amounts respectively detected at multiple time points.

Furthermore, the output information transmitter 309 transmits the generated output information to the receiving device 20 using the communicator 33.

### (operation)

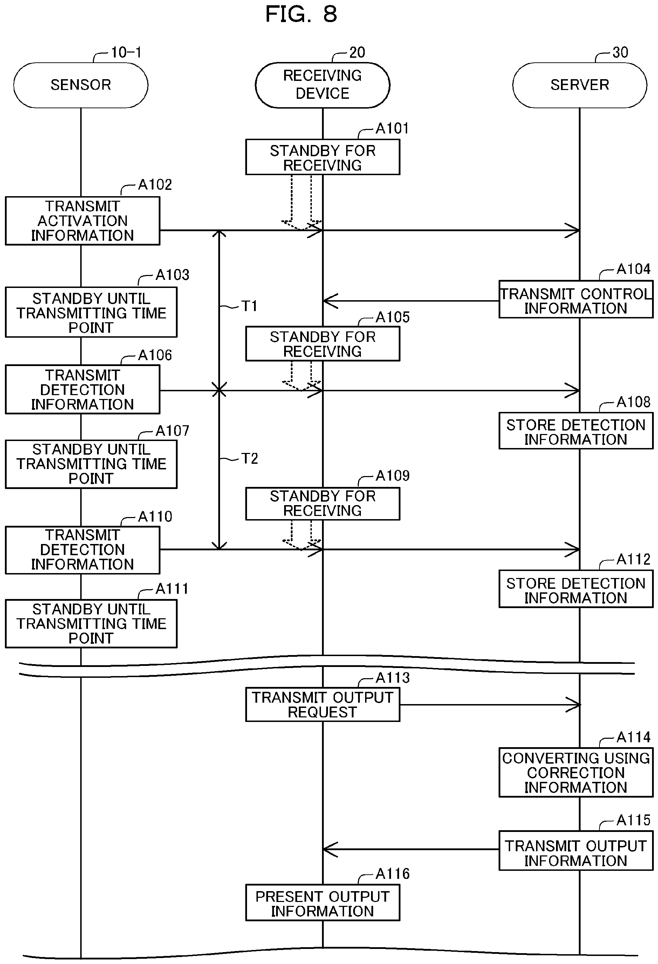

Next, description will now be made in relation to operation in the detecting system 1 by referring to FIGs. 8-14.

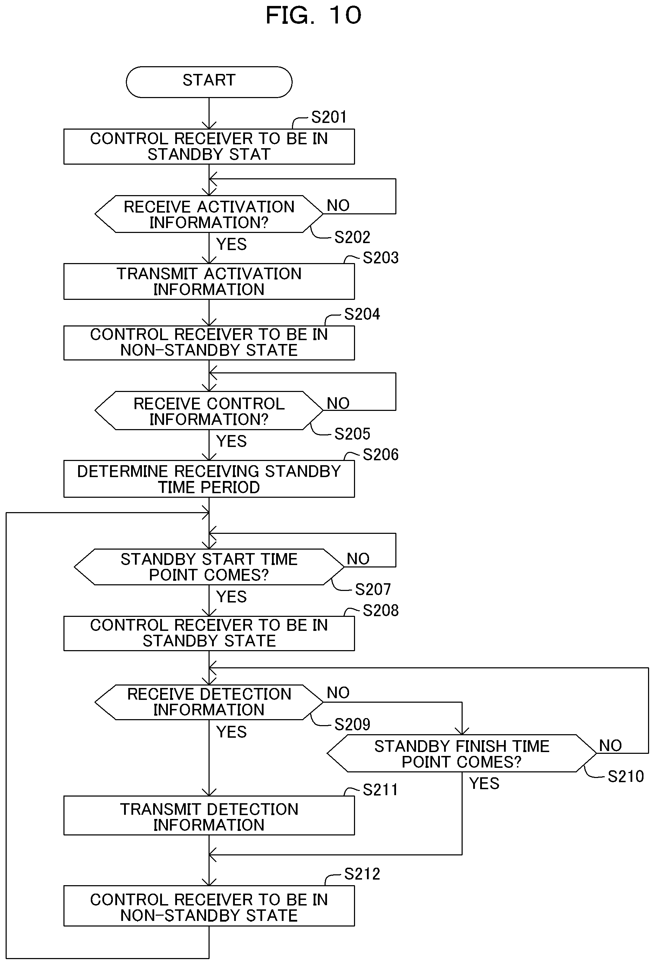

First of all, after being activated, the receiving device 20 controls the receiver 23 to be in the standby state (step A101 of FIG. 8 and step S201 of FIG. 10). The receiving device 20 waits (is on standby) until receiving activation information from the sensor 10-n ("No" route of step S202 of FIG. 10) .

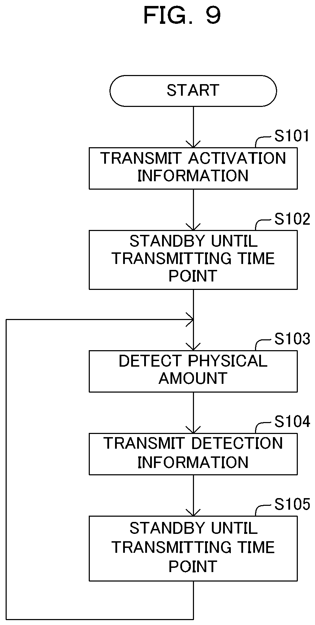

The sensor 10-1 is introduced into the living body through the mouth. Thereby, the sensor 10-1 detects the start of using the sensor 10-1. Consequently, the sensor 10-1 transmits the activation information including the identification information stored therein to the receiving device 20 (step A102 of FIG. 8 and step S101 of FIG. 9).

Then the sensor 10-1 waits until the transmitting time point represented by the transmitting time-point information stored therein comes (step A103 of FIG. 8 and step S102 of FIG. 9).

On the other hand, the receiving device 20 receives the activation information from the sensor 10-1. Thereby, the receiving device 20 makes the "Yes" determination in step S202 of FIG. 10, and transmits the received activation information to the server 30 (step A102 of FIG. 8 and step S203 of FIG. 10). Then the receiving device 20 controls the receiver 23 to be in non-standby state (step S204 of FIG. 10). The receiving device 20 waits until receiving the control information from the server 30 ("No" route of step S205 of FIG. 10).

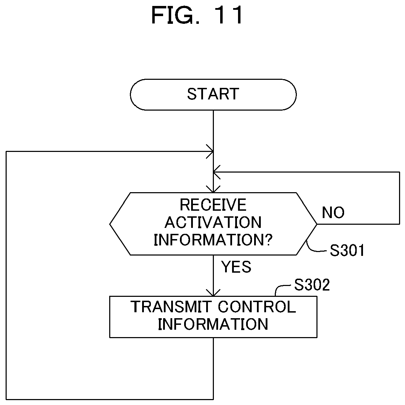

After being activated, the server 30 waits until the server 30 receives the activation information from the receiving device 20 ("No" route of step S301 of FIG. 11). Upon receipt of the activation information from the receiving device 20, the server 30 makes the "Yes" determination in step S301 of FIG. 11.

Next, the server 30 obtains the transmitting time-point information stored therein in association with the type identification information included in the identification information included in the received activation information. The server 30 transmits the control information including the obtained transmitting time-point information and the identification information included in the received activation information to the receiving device 20 (step A104 of FIG. 8 and and step S302 of FIG. 11). Next, the server 30 returns to step S301 of FIG. 11 and repeats the process of steps S301 and S302.

Thereby, the receiving device 20 receives the control information from the server 30. Consequently, the receiving device 20 makes the "Yes" determination in step S205 of FIG. 10, and determines multiple standby time periods based on the received control information and the time point at which the receiving device 20 receives the activation information (step S206 of FIG. 10).

Then the receiving device 20 waits until the time point (in other words, the standby start time point) at which the receiving standby time period that starts the earliest among the determined multiple receiving standby time periods after the current time point comes ("No" route of step S207 of FIG. 10).

Next, when the standby start time point comes, the receiving device 20 makes the "Yes" determination in step S207 of FIG. 10, and controls the receiver 23 to be in the standby state (step A105 of FIG. 8 and step S208 of FIG. 10).

Then the receiving device 20 determines whether detection information is received from the sensor 10-1 (step S209 of FIG. 10). When not receiving detection information from the sensor 10-1, the receiving device 20 makes the "No" determination in step S209 of FIG. 10.

The receiving device 20 determines whether the time point (in other words, the standby finish time point) at which the receiving time period that finishes the earliest among the determined multiple receiving standby time periods after the current time point comes (step S210 of FIG. 10). When the standby finish time point does not come, the receiving device 20 makes the "No" determination in step S210 and returns to step S209 to repeat the process of steps S209 and 210.

When the transmitting time point represented by the transmitting time point information stored in the sensor 10-1 comes, the sensor 10-1 detects a physical amount by converting the physical amount into a signal value (step S103 of FIG. 9). Then the sensor 10-1 transmits detection information representing the signal value obtained by converting the physical value to the receiving device 20 (step A106 of FIG. 8 and step S104 of FIG. 9).

Then the sensor 10-1 waits until the next transmitting time point represented by the transmitting time-point information stored therein comes (step A107 of FIG. 8 and step S105 of FIG. 9). After that, the sensor 10-1 returns to step S103 and repeats the process of step S103 to step S105.

On the other hand, the receiving device 20 receives the detection information from the sensor 10-1. This causes the receiving device 20 to make the "Yes" determination in step S209 of FIG. 10, and transmits the received detection information to the server 30 (step A106 of FIG. 8 and step S211 of FIG. 10). The receiving device 20 controls the receiver 23 to be in the non-standby state (step S212 of FIG. 10). Then the receiving device 20 returns to step S207 and repeats the process of step S207 to step S212.

When the receiving device 20 does not receive detection information until the standby finish time point comes after the standby start time point comes, the receiver makes the "Yes" determination in step S210 of FIG. 10 and proceeds to step S212 and the subsequent steps without carrying out the process of step S211.

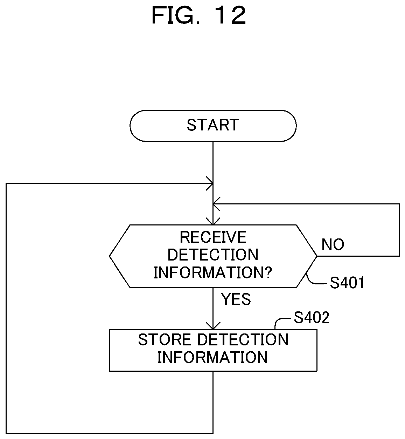

Here, the server 30, after being activated, waits until receiving detection information from the receiving device 20 ("No" route of step S401 of FIG. 12). Upon receipt of the detection information from the receiving device 20, the server 30 makes the "Yes" determination in step S401 of FIG. 12.

Then the server 30 stores therein the received detection information (step A108 of FIG. 8 and step S402 of FIG. 12). Next, the server 30 returns to step S401 of FIG. 12 and repeats the process of steps S401 and S402.

Then the detecting system 1 carries out the process of step A109 to A112 like the process of step A105 to A108.

After that, the detecting system 1 repeats the process the same as the process of step A105 to A108.

In this example, the time period T1 between the transmission of the activation information in step A102 and the transmission of the detection information in step A106 of FIG. 8 corresponds to the shortest transmitting standby time among the multiple transmitting standby times represented by the transmitting time-point information. In this example, the sum of the time period T1 and a time period T2 between the transmission of the detection information in step A106 and the transmission of the (next) detection information in step A110 of FIG. 8 corresponds to the second shortest transmitting standby time among the multiple transmitting standby times represented by the transmitting time-point information.

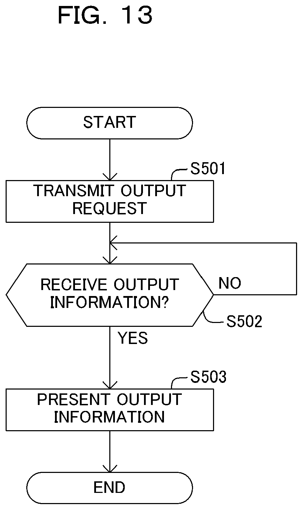

Further, when the user of the receiving device 20 inputs predetermined information via the I/O device 25 into the receiving device 20, the receiving device 20 transmits an output request including the identification information stored therein to the server 30 (step A113 of FIG. 8 and step S501 of FIG. 13). Then the receiving device 20 waits until receiving output information from the server 30 ("No" route of step S502 of FIG. 13).

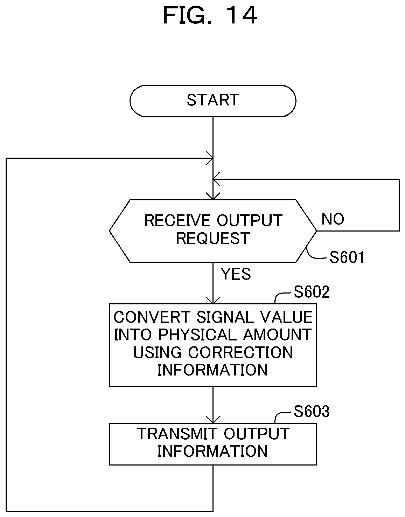

After being activated, the server 30 waits until receiving an output request from the receiving device 20 ("No" route of step S601 of FIG. 14). After that, upon receipt of the output request from the receiving device 20, the server 30 makes the "Yes" determination in step S601 of FIG. 14.

Next, the server 30 obtains the detection information including the identification information included in the received output request from multiple pieces of detection information stored therein. Furthermore, the sever 30 obtains correction information stored therein in association with the type identification information included in the identification information included in the received output request.

Then the server 30 corrects the relationship between the signal value represented by the obtained detection information and the physical amount using the obtained correction information, and converts the signal value represented by the obtained detection information into the physical amount in accordance with the corrected relationship (step A114 of FIG. 8 and step S602 of FIG. 14).

Next, the server 30 generates output information based on the physical amount obtained by the conversion, and transmits the generated output information to the receiving device 20 (step A115 of FIG. 8 and step S603 of FIG. 14). After that, the server 30 returns to step S601 and repeats the process of steps S601 to S603.

The receiving device 20 responsively receives the output information from the server 30. Accordingly, the receiving device 20 makes the "Yes" determination in step S502 of FIG 13 and outputs (presents) the received output information via the I/O device 25 (step A116 of FIG 8 and step S503 of FIG. 13). After that, the receiving device 20 finishes the process of FIG. 13.

As described above, in the detecting system 1 of the first embodiment, the sensor 10-n is introduced into a living body through the mouth, and transmits the time-point related information (in this example, the activation information) associated with multiple time points different from one another. Furthermore, the sensor 10-n detects the physical amount in the living body and transmits detection information representing the detected physical amount at each of the above multiple time points.

In addition, the receiving device 20 receives the time-point related information transmitted by the sensor 10-n, and is on standby for receiving the detection information transmitted by the sensor 10-n on the basis of the multiple time points associated with the received time-point related information.

This can further reduce the electric power that the sensor 10-n consumes as compared with cases where control information is transmitted from the receiving device 20 to the sensor 10-n. Further, even if the time point at which the detection information is transmitted from the sensor 10-n is different with the sensors 10, the receiving device 20 can be appropriately controlled to be on standby for receiving the detection information depending on the sensor 10-n introduced in a living body, so that the electric power that the receiving device 20 consumes can be reduced.

Accordingly, the detecting system 1 can reduce the consumed electric power.

Further, in the detecting system 1 according to the first embodiment, the multiple time points at which the detection information is transmitted is set for each type of sensor 10. In addition, the detecting system 1 stores a type of sensor 10 and multiple time points set for the type of sensor 10 in association with each other. The time-point related information represents the type of sensor 10.

In addition, the receiving device 20 is on standby for receiving the detection information transmitted by the sensor 10-n on the basis of the multiple time points stored in association with the type represented by the received time-point related information and the time point at which the time-point related information is received or transmitted.

A time point at which the sensor 10 transmits the detection information may be different with types of sensor 10. Accordingly, the detecting system 1 can appropriately control the receiving device 20 to be on standby for receiving the detection information for each type of sensor 10-n, so that the electric power consumed by the receiving device 20 can be reduced.

Further, in the detecting system 1 of the first embodiment, the sensor 10-n starts transmission of detection information after transmitting the time-point related information.

This can further reduce the electric power consumed by the receiving device 20 as compared with cases where the detection information is transmitted before the time-point related information is transmitted.

Further, in the detecting system 1 of the first embodiment, the sensor 10-n detects a physical amount by converting the physical amount into a signal value. Further, the detection information represents the signal value. In addition, the time-point related information identifies the sensor 10-n among the multiple sensors 10-1, ..., and 10-N.

Furthermore, the detecting system 1 stores therein a sensor 10-n and the correction information for correcting the relationship between a signal value and a physical amount in the sensor 10-n in association with each other. In addition, the detecting system 1 corrects the relationship between a signal value represented by the received detection information and the physical amount on the basis of the correction information stored in association with the sensor 10-n identified by the received time-point related information.

Here, the relationship between a physical amount and a signal value may sometimes be different with sensors 10 because of manufacturing variation to make it difficult to precisely detect the physical amount.

As a solution to the above, the detecting system 1 can correct the relationship between a signal value and a physical amount on the basis of the correction information for each sensor 10. This allows to precisely detect the physical amount.

The receiving device 20 may be configured by multiple devices communicably connected to one another. In this case, examples of at least one of the multiple devices constituting the receiving device 20 may be a mobile phone, a smartphone, or a personal computer.

### <second embodiment>

Next, description will now be made in relation to a detecting system according to a second embodiment. A detecting system of the second embodiment is different from the detecting system 1 of the first embodiment in the point of warning against cases where multiple sensors are introduced into a living body within a predetermined time period. Hereinafter, description will focus on the difference. Like reference numbers designate the same or similar devices and elements between the first embodiment and the second embodiment.

### (function)

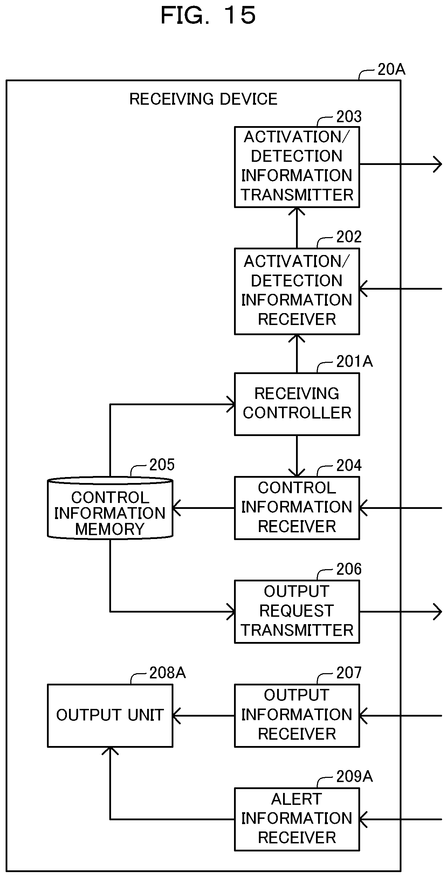

As illustrated in FIG. 15, the function of a receiving device 20A of the second embodiment includes a receiving controller 201A and an output unit 208A respectively in place of the receiving controller 201 and the output unit 208 included in the receiving device 20 of the first embodiment. The function of the receiving device 20A further includes an alert information receiver 209A.

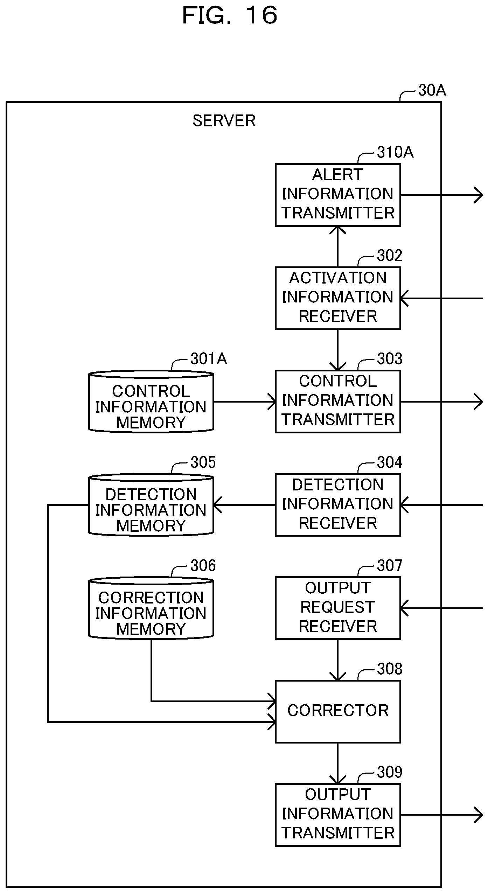

As illustrated in FIG. 16, the function of a server 30A of the second embodiment includes a control information memory 301A in place of the control information memory 301 included in the server 30 of the first embodiment. The function of the server 30A further includes an alert information transmitter 310A.

The control information memory 301A stores, for each type of sensor 10, the type identification information that identifies the type, transmitting time-point information representing multiple transmitting time points set for the type of sensor 10, and activation information standby time information representing an activation information standby time set for the type of sensor 10 in association with one another using the memory 32.

In this example, the control information includes the identification information, the transmitting time-point information, and the activation information standby time information.

The activation information standby time is a time between a time point at which the receiving device 20A first receives the activation information and a time point at which the receiving device 20A completes switching of the state of the receiver 23 from the standby state to the non-standby state. In other words, the activation information standby time is a time between a time point at which the receiving device 20A receives the activation information transmitted by a sensor 10-n and a time point at which the receiving device 20A terminates the standby for receiving activation information transmitted by another sensor 10-m. The symbol m represents an integer different from n between 1 to N.

The receiving controller 201A controls the receiver 23 to be in the standby state or the non-standby state.

When the receiving device 20A is activated, the receiving controller 201A controls the state of the receiver 23 to be in the standby state. When the activation/detection information receiver 202 receives the activation information, the receiving controller 201A keeps the receiver 23 to be in the standby state.

The receiving controller 201A determines multiple receiving standby time periods on the basis of the control information stored in the control information memory 205, and the time point at which the activation/detection information receiver 202 receives the activation information (i.e., the activation information received time point).

The receiving controller 201A may use a time point at which the activation information is transmitted as the time point at which the activation information is received. In this case, the activation information may include therein information representing a time point at which the activation information is transmitted.

In this example, the multiple receiving standby time periods determined by the receiving controller 201A include a single activation information receiving standby time period and multiple detection information receiving standby time periods. The activation information receiving standby time period is a time period from the activation information received time point to the time point at which the activation information standby time represented by the activation information standby time information included in the control information has passed since the activation information received time point. The multiple detection information receiving standby time periods respectively include multiple time points (i.e., estimated reception time points) at which multiple transmitting standby times represented by the transmitting time-point information included in the control information have respectively passed since the activation information received time point. In this example, the length of the detection information receiving standby time period is determined in advance.

The time point at which the receiving standby time period starts may be referred to as a standby start time point while the time point at which the receiving standby time period finishes may be referred to as a standby finish time point. In this example, determination of the receiving standby time period includes determination of the standby start time point and determination of the standby finish time point.

For example, in the detection information receiving standby time period, the length of from the standby start time point to the estimated reception time point may be the same as the length of from the estimated reception time point to the standby finish time point. Alternatively, in the detection information receiving standby time period, the length of from the standby start time point to the estimated reception time point may be different from the length of from the estimated reception time point to the standby finish time point.

The receiving controller 201A controls the receiver 23 to be in the standby state each time the determined standby start time point comes.

Each time the activation/detection information receiver 202 receives the detection information, the receiving controller 201A controls the receiver 23 to be in the non-standby state. Furthermore, each time the determined standby finish time point comes, the receiving controller 201A controls the receiver 23 to be in the non-standby state.

In cases where the activation information receiver 302 receives multiple pieces of activation information from the receiving device 20A within the activation information receiving standby time period, the alert information transmitter 310A transmits warning information to the receiving device 20A using the communicator 33. In this example, receiving multiple pieces of activation information from the receiving device 20A within the activation information receiving standby time period corresponds to cases where multiple different sensors 10-n, 10-m,... respectively transmit different pieces of activation information within the activation information receiving standby time period.

The alert information warns the user of the receiving device 20A. For example, the alert information may represent that multiple sensors 10-n, 10-m,... are being used at the same time or may represent that abnormality is occurring.

The alert information receiver 209A receives the alert information transmitted by the server 30A using the communicator 24.

The output unit 208A outputs, when the output information receiver 207 receives output information, the received output information using the I/O device 25. Furthermore, the output unit 208A outputs, when the alert information receiver 209A receives the alert information, the received alert information using the I/O device 25.

### (operation)

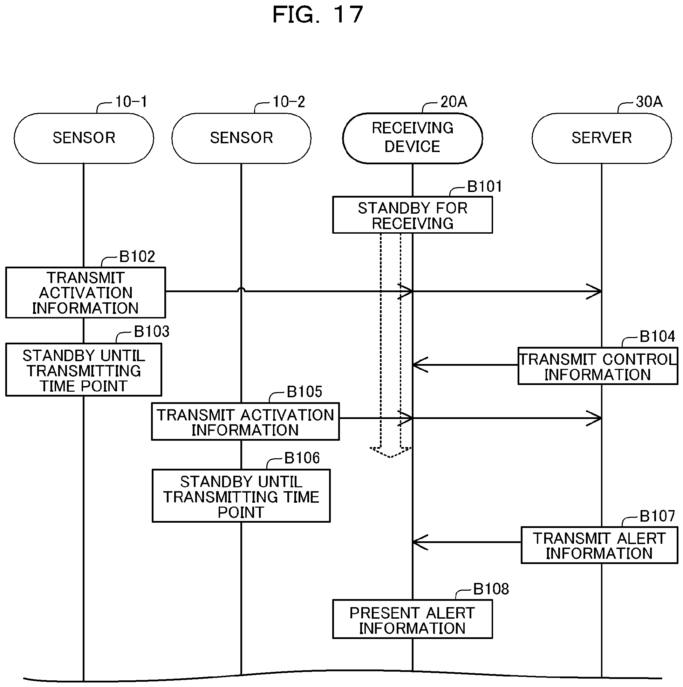

Next, description will now be made in relation to the detecting system 1 of the second embodiment by referring to FIGs. 17-20.

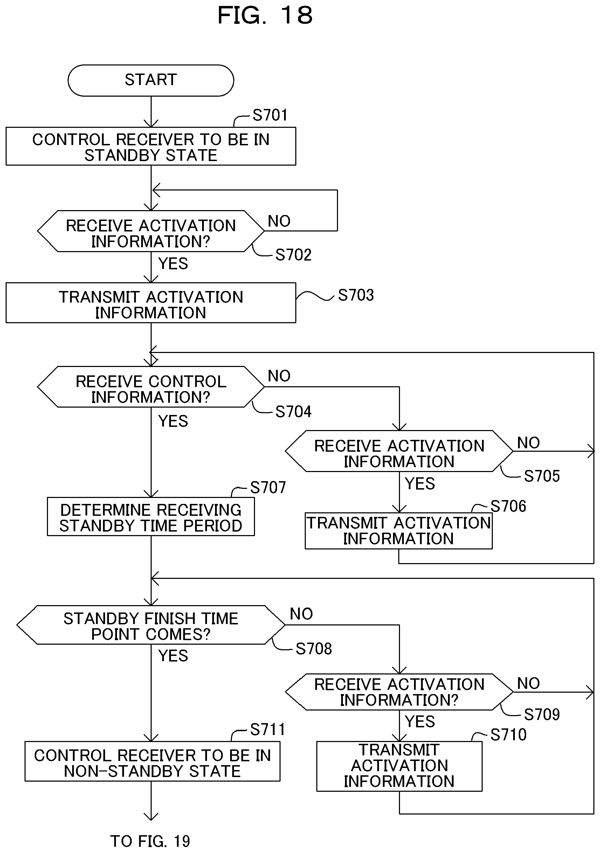

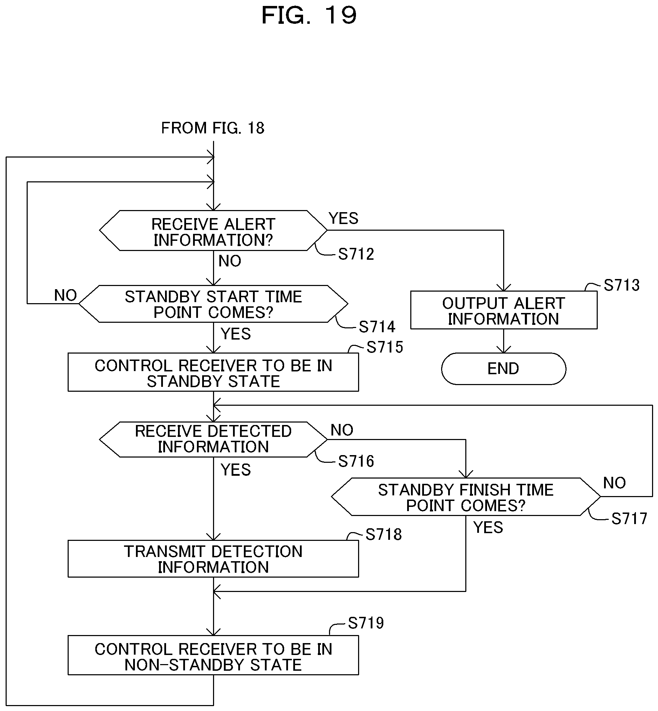

The receiving device 20A of the second embodiment is different from the receiving device 20 of the first embodiment in the point of performing the process of FIGs. 18 and 19 in place of the process of FIG. 10.

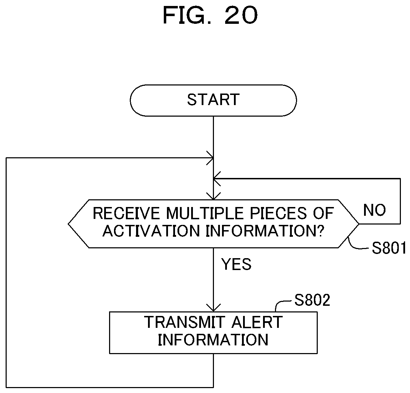

The server 30A of the second embodiment is different from the server 30 of the first embodiment in the point of performing the process of FIG. 20 in addition to the process performed by the server 30.

This example assumes a case where a sensor 10-2 is introduced into a living body through the mouth before the activation information standby time represented by the activation information standby time information set for the sensor 10-1 passes since the sensor 10-1 is introduced into the living body through the mouth.

First of all, after being activated, the receiving device 20A controls the receiver 23 to be in the standby state (step B101 of FIG. 17 and step S701 of FIG. 18). Then the receiving device 20A waits until receiving the activation information from the sensor 10-n ("No" route of step S702 of FIG. 18).

The sensor 10-1 is introduced into the living body through the mouth. Thereby, the sensor 10-1 detects the start of using the sensor 10-1. Consequently, the sensor 10-1 transmits the activation information including the identification information stored therein to the receiving device 20A (step B102 of FIG. 17 and step S101 of FIG. 9).

Then the sensor 10-1 waits until the transmitting time point represented by the transmitting time-point information stored therein comes (step B103 of FIG. 17 and step S102 of FIG. 9).

On the other hand, the receiving device 20A receives the activation information from the sensor 10-1. Thereby, the receiving device 20A makes the "Yes" determination in step S702 of FIG. 18, and transmits the received activation information to the server 30A (step B102 of FIG. 17 and step S703 of FIG. 18).

Then the receiving device 20A determines whether the receiving device 20A receives the control information from the server 30A (step S704 of FIG. 18). When not receiving the control information from the server 30A, the receiving device 20A makes the "No" determination in step S704 of FIG. 18.

Next, the receiving device 20A determines whether the receiving device 20A receives, from the sensor 10-m (in this example, the sensor 10-2), activation information different from the activation information received in step S702 (step S705 of FIG. 18).

When not receiving the activation information from the sensor 10-m, the receiving device 20A makes the "No" determination in step S705 of FIG. 18, returns to step S704 to repeat the process of steps S704 to S705.

When being activated, the server 30A waits until the server 30 receives the activation information from the receiving device 20A ("No" route of step S301 of FIG. 11). Upon receipt of the activation information from the receiving device 20A, the server 30A makes the "Yes" determination in step S301 of FIG. 11.

Next, the server 30A obtains the transmitting time-point information and the activation information standby time information that are stored in association with the type identification information included in the identification information included in the received activation information. The server 30A transmits the control information including the obtained transmitting time-point information, the obtained activation information standby time information, and the identification information included in the received activation information to the receiving device 20A (step B104 of FIG. 17 and step S302 of FIG. 11). Next, the server 30A returns to step S301 of FIG. 11 and repeats the process of steps S301 and S302.

Thereby, the receiving device 20A receives the control information from the server 30A. Consequently, the receiving device 20A makes the "Yes" determination in step S704 of FIG. 18, and determines multiple receiving standby time periods based on the received control information and the time point of receiving the activation information (step S707 of FIG. 18).

Then, the receiving device 20A determines whether a time point (i.e., the standby finish time point) at which the activation information receiving standby time period among the determined multiple receiving standby time periods ends comes (step S708 of FIG. 18). When the standby finish time point has not come yet, the receiving device 20A makes the "No" determination in step S708 of FIG. 18, and further determines whether the receiving device 20A receives, from the sensor 10-m (in this example, sensor 10-2), activation information different from the activation information received in step S702 (step S709 of FIG. 18).

When not receiving the activation information from the sensor 10-m, the receiving device 20A makes the "No" determination in step S709 of FIG. 18 and returns to step S708 to repeat the process of step S708 and S709.

The sensor 10-2 is introduced into the living body through the mouth. Thereby the sensor 10-2 detects the start of using the sensor 10-2. Consequently, the sensor 10-2 transmits the activation information including the identification information stored therein to the receiving device 20A (step B105 of FIG. 17 and step S101 of FIG. 9).

Then, the sensor 10-2 waits until the transmitting time point represented by the transmitting time-point information stored therein comes (step B106 of FIG. 17 and step S102 of FIG. 9).

On the other hand, the receiving device 20A receives the activation information from the sensor 10-2. Consequently, the receiving device 20A makes the "Yes" determination in step S709 of FIG. 18, and transmits the received activation information to the server 30A (step B105 of FIG. 17 and step S710 of FIG. 18).

When the standby finish time point comes, the receiving device 20A makes the "Yes" determination in step S708 of FIG. 18, and controls the receiver 23 to be in the non-standby state (step S711 of FIG. 18).

Next, the receiving device 20A determines whether the receiving device 20A receives alert information from the server 30A (step S712 of FIG. 19). When not receiving the alert information from the server 30A, the receiving device 20A makes the "No" determination in step S712 of FIG. 19.

Then the receiving device 20A determines whether the time point (in other words, the standby start time point), at which the detection information receiving standby time period that starts the earliest after the current time point among the determined multiple detection information receiving standby time periods starts, comes (step S714 of FIG. 19). When the standby start time point has not come yet, the receiving device 20A makes the "No" determination in step S714 of FIG. 19 and returns to step S712 to repeat the process of step S712 and S714.

After being activated, the server 30A waits until receiving multiple pieces of the activation information from the receiving device 20A during the activation information receiving standby time period ("No" route of step S801 of FIG. 20). When receiving multiple pieces of the activation information from the receiving device 20A during the activation information receiving standby time period, the server 30A makes the "Yes" determination in step S801 of FIG. 20.

Next, the server 30A transmits the alert information to the receiving device 20A (step B107 of FIG. 17 and step S802 of FIG. 20). Next, the server 30A returns to step S801 of FIG. 20 and repeats the process of steps S801 and S802.

Thereby, the receiving device 20A receives the alert information from the server 30A. Consequently, the receiving device 20A makes the "Yes" determination in step S712 of FIG. 19, and then presents the received alert information via the I/O device 25 (step B108 of FIG. 17 and step S713 of FIG. 19). After that, the receiving device 20A finishes the process of FIG. 19.

When the receiving device 20A does not receive the alert information until the standby start time point comes, the receiving device 20A makes the "Yes" determination in step S714 of FIG. 19 and executes the process of steps S715 to S719 likewise the process of steps S208 to S212 of FIG. 10. After that, the receiving device 20A returns to step S712 and repeats the process of steps S712 to S719.

When receiving activation information from the sensor 10-2 before receiving the control information, the receiving device 20A makes the "Yes" determination in step S705 of FIG. 18, and transmits the received activation information to the server 30A (step S706 of FIG. 18). Then the receiving device 20A returns to step S704.

As described above, when receiving the alert information, the receiving device 20A of this example stops being standby for receiving detection information. Alternatively, when receiving the alert information, the receiving device 20A may be on standby for receiving the detection information. In this alternative, after performing the process of step S713 of FIG. 19, the receiving device 20A may proceed to step S714 of FIG. 19.

Further alternatively, when receiving the alert information, the receiving device 20A may finish the process of FIG. 19 without presenting the alert information. This means that the receiving device 20A may stop being standby for receiving the detection information in place of presenting the alert information.

Further alternatively, when receiving the alert information, the receiving device 20A may be on standby for receiving the detection information and also stop transmitting the output request or presenting (outputting) the output information in place of or in addition to presenting the alert information. In this modification, transmitting the output request or presenting the output information corresponds to a process on the received detection information.

Alternatively, in the detecting system 1, the receiving device 20A may be on standby for receiving the detection information and also the server 30A may store therein the detection information in association with predetermined flag information in place of or in addition to presenting the alert information.

As described above, the detecting system 1 of the second embodiment can bring the same effects and advantages as the first embodiment.

Furthermore, in the detecting system 1 of the second embodiment, the receiving device 20A is on standby for receiving the time-point related information transmitted by another sensor 10-m for a time period from a first time point at which the receiving device 20A receives the time-point related information (in this example, activation information) to a second time point at which a standby time (in this example, activation information standby time) associated with the received time-point related information passes since the first time point.

Here, multiple sensors 10-n, 10-m, ... mistakenly introduced in a living body through a mouth may transmit multiple pieces of time-point related information in a considerable short time period, respectively. In this case, when the sensors 10 respectively have different clock frequencies, multiple pieces of detection information may be simultaneously transmitted by multiple sensors 10-n and 10-m,..., after a predetermined time period has passed. This case has a high possibility that the detection information received by the receiving device includes an error.

As a solution to the above, the detecting system 1 can detect that multiple pieces of time-point related information have been transmitted by the multiple sensors 10-n, 10-m,... introduced in the living body within the standby time. Accordingly, the detecting system 1 can warn the user of the detecting system 1.

Accordingly, the detecting system 1 can suppress the use of the detection information without making the user recognize that the detection information has high possibility of including an error.

Furthermore, in the detecting system 1 of the second embodiment, the standby time (in this example, activation information standby time) is set for each type of sensor 10. The detecting system 1 stores each type of sensor 10 and the standby time set for the type of sensor 10 in association with each other.

In addition, the time-point related information represents the type of sensor 10. Further, the receiving device 20A is on standby for receiving the time-point related information transmitted by another sensor 10-m during a time period from a first time point at which the time-point related information is received to a second time point at which the standby time stored in association with the type represented by the received time-point related information passes since the first time point.

Time point at which a sensor 10 transmits detection information may be different with types of sensor 10. Accordingly, in cases where multiple sensors 10-n, 10-m,... are introduced into a living body, the possibility of the presence of an error in the detection information received by the receiving device 20A strongly correlates with the type of sensor 10.

Accordingly, the detecting system 1 can appropriately detect multiple pieces of time-point related information respectively transmitted from multiple sensors 10-n, 10-m,... introduced into the living body by considering the type of each sensor 10. Consequently, the detecting system 1 can suppress the use of the detection information without making the user recognize that the detection information has high possibility of including an error.

Furthermore, in cases where the receiving device 20A receives the time-point related information transmitted by another sensor 10-m within a certain time period (in this example, activation information receiving standby time period), the detecting system 1 of the second embodiment warns the user of the detecting system 1.

This makes it possible to warn the user of the detecting system 1. Accordingly, the detecting system 1 can suppress the use of the detection information without making the user recognize that the detection information has high possibility of including an error.

Here, the server 30A may transmit the alert information when multiple pieces of the activation information received from the receiving device 20A during the activation information receiving standby time period satisfy a predetermined alert condition, but may stop the transmission of the alert information when the multiple pieces of activation information do not satisfy the alert condition.

An example of the alert condition is that at least two of the multiple pieces of activation information include identification information including the same type identification information.

Another example of the alert information is that at least one of the multiple transmitting time points represented by the transmitting time-point information stored in association with the type identification information included in the identification information included in each piece of the activation information is common to at least two of the multiple pieces of the activation information.

In cases where the time point at which a sensor 10-n transmits detection information coincides with the time point at which another sensor 10-m transmits detection information, multiple pieces of detection information are simultaneously transmitted from the multiple sensors 10-n and 10-m. This case has high possibility that the detection information received by the receiving device 20A includes an error. Accordingly, this modification can surely suppress the use of the detection information without making the user recognize that the detection information has high possibility of including an error in the detecting system 1.

Alternatively, in place of the server 30A, the receiving device 20A may determine whether the multiple pieces of the activation information are received within the activation information receiving standby time period in the detecting system 1. In this case, the server 30A may omit the transmission of the alert information. Also in this case, the receiving device 20A may determine whether the alert information will be output on the basis of multiple pieces of control information that the server 30A transmits in response to the transmission of the multiple pieces of activation information received within the activation information receiving standby time period.

Further, the present invention should by no means to be limited to the foregoing embodiments. For example, various modification and changes that those ordinary skilled in the art understand can be added to the above embodiment without departing from the spirit of the present invention. For example, a combination of the above embodiments and modification can be adopted as an additional modification to the embodiments within the scope of the present invention.

### REFERENCE NUMERAL LIST

- 1: detecting system
- 10: sensor
- 11: controller
- 12: memory
- 13: detector
- 14: transmitter
- 101: identification information memory
- 102: activation information transmitter
- 103: detection unit
- 104: detection information transmitter
- 20,20A: receiving device
- 21: controller
- 22: memory
- 23: receiver
- 24: communicator
- 25: I/O device
- 201,201A: receiving controller
- 202: activation/detection information receiver
- 203: activation/detection information transmitter
- 204: control information receiver
- 205: control information memory
- 206: output request transmitter
- 207: output information receiver
- 208,208A: output unit
- 209A: alert information receiver
- 30,30A: server
- 31: controller
- 32: memory
- 33: communicator
- 301,301A: control information memory
- 302: activation information receiver
- 303: control information transmitter
- 304: detection information receiver
- 305: detection information memory
- 306: correction information memory
- 307: output request receiver
- 308: corrector
- 309: output information transmitter
- 310A: alert information transmitter
- BU1, BU2, BU3: bus
- NW: network

## Claims

1. A detecting system comprising:
a sensor that is introduced into a living body through a mouth of the living body, that transmits time-point related information associated with a plurality of time points different from one another, that detects a physical amount in the living body, and that transmits detection information representing the detected physical amount at each of the plurality of time points; and
a receiving device that receives the time-point related information transmitted by the sensor and that is on standby for receiving the detection information transmitted by the sensor on the basis of the plurality of time points associated with the received time-point related information.

2. The detecting system according to claim 1, wherein the receiving device is on standby for receiving other time-point related information transmitted by another sensor during a time period from a first time point at which the receiving device receives the time-point related information to a second time point at which a standby time associated with the received time-point related information passes since the first time point.

3. The detecting system according to claim 1 or 2, wherein:
the plurality of time points are set for a type of sensor;
the detecting system stores therein a type of sensor and a plurality of time points set for the type of the sensor in association with each other;
the time-point related information represents the type of the sensor; and
the receiving device is on standby for receiving the detection information transmitted by the sensor on the basis of the plurality of time points stored in association with the type represented by the received time-point related information and a time point at which the receiving device receives the time-point related information.

4. The detecting system according to any one of claims 1-3, wherein:
the standby time is set for a type of sensor;
the detecting system stores therein a type of sensor and the standby time set for the type of the sensor in association with each other;
the time-point related information represents the type of the sensor; and
the receiving device is on standby for receiving other time-point related information transmitted by another sensor during a time period from a first time point at which the receiving device receives the time-point related information to a second time point at which the standby time stored in association with the type represented by the received time-point related information passes since the first time point.

5. The detecting system according to claim 2 or 4, wherein
when the receiving device receives the time-point related information transmitted by the other sensor during the time period, the receiving device carries out at least one selected from a group consisting of warning against a user of the detecting system, stopping the standby for receiving the detection information, and stopping a process on the received detection information.

6. The detecting system according to claim 2 or 4, wherein
when the receiving device receives the time-point related information transmitted by the other sensor during the time period and also when at least one of the plurality of the time points associated with the time-point related information received from the sensor coincides with at least one of the plurality of the time points associated with the time-point related information received from the other sensor,
the receiving device carries out at least one selected from a group consisting of warning against a user of the detecting system, stopping the standby for receiving the detection information, and stopping a process on the received detection information.

7. The detecting system according to any one of claims 1-6, wherein the sensor starts transmitting the detection information after transmitting the time-point related information.

8. The detecting system according to any one of claims 1-7, wherein:
the sensor detects the physical amount by converting the physical amount into a signal value;
the detection information represents the signal value;
the time-point related information identifies the sensor among a plurality of sensors; and
the detecting system stores therein a sensor and correction information in association with each other, the correction information being used for correcting relationship between the signal value and the physical amount in the sensor, and corrects the relationship between a signal value represented by the received detection information and the physical amount using the correction information stored in association with the sensor identified by the received time-point related information.

9. A receiving device comprising:
a receiver that receives time-point related information and detection information that are transmitted by a sensor(10), the sensor being introduced into a living body through a mouth of the living body, detecting a physical amount in the living body, and transmitting the detection information representing the detected physical amount at each of a plurality of time points; and
a controller that controls the receiver to be on standby for receiving the detection information transmitted by the sensor on the basis of the plurality of time points associated with the received time-point related information.

10. The receiving device according to claim 9, wherein the controller controls the receiver to be on standby for receiving other time-point related information transmitted by another sensor during a time period from a first time point at which the receiving device receives the time-point related information to a second time point at which a standby time associated with the received time-point related information passes since the first time point.

11. A method for detecting comprising:
introducing a sensor into a living body through a mouth of the living body;
at the sensor,
transmitting time-point related information associated with a plurality of time points different from one another;
at a receiving device,
receiving the time-point related information transmitted by the sensor;
at the sensor,
detecting a physical amount in the living body;
at the sensor,
transmitting detection information representing the detected physical amount at each of the plurality of time points; and
at the receiving device,
being on standby for receiving the detection information transmitted by the sensor on the basis of the plurality of time points associated with the received time-point related information.

12. The method according to claim 11, further comprising
at the receiving device,
being on standby for receiving other time-point related information transmitted by another sensor during a time period from a first time point at which the receiving device receives the time-point related information to a second time point at which a standby time associated with the received time-point related information passes since the first time point.
